# EUROPÄISCHE PATENTSCHRIFT

(11) **EP 2 117 997 B1**
(45) Veröffentlichungstag und Bekanntmachung des Hinweises auf die Patenterteilung: **03.10.2012**
(21) Anmeldenummer: 08708519.7
(22) Anmeldetag: 31.01.2008
(51) Int. Cl.: C07C 5/48, C01B 3/36, B01J 4/00, F23L 7/00, F23C 6/04

(54) **VERFAHREN ZUR BEREITSTELLUNG EINES SAUERSTOFF ENTHALTENDEN GASSTROMES FÜR DIE ENDOTHERME UMSETZUNG EINES AUSGANGSSTROMES, ENTHALTEND EINEN ODER MEHRERE KOHLENWASSERSTOFFE**
METHOD FOR PROVIDING A GAS FLOW COMPRISING OXYGEN FOR THE ENDOTHERMIC REACTION OF A STARTING FLOW COMPRISING ONE OR MORE HYDROCARBONS
PROCÉDÉ POUR FOURNIR UN FLUX DE GAZ CONTENANT DE L'OXYGÈNE POUR LA RÉACTION ENDOTHERMIQUE D'UN FLUX DE DÉPART CONTENANT UN OU PLUSIEURS HYDROCARBURES

(30) Priorität: 06.02.2007 EP 07101829
(43) Veröffentlichungstag der Anmeldung: 18.11.2009
(73) Patentinhaber: BASF SE, 67056 Ludwigshafen (DE)
(72) Erfinder: OLBERT, Gerhard, 69221 Dossenheim (DE); CORR, Franz, 67067 Ludwigshafen (DE); CRONE, Sven, 67117 Limburgerhof (DE)
(86) Internationale Anmeldenummer: PCT/EP2008/051208
(87) Internationale Veröffentlichungsnummer: WO 2008/095860

(56) Entgegenhaltungen:
- EP-A- 0 406 071
- WO-A-00/06948
- WO-A-99/11571
- WO-A-2006/119812
- DE-A1-102004 024 957
- GB-A- 776 106
- GB-A- 1 394 988
- US-A- 5 087 270
- US-A- 5 784 876

## Beschreibung

Die Erfindung betrifft ein Verfahren zur Bereitstellung eines Sauerstoff enthaltenden Gasstromes für die endotherme Umsetzung eines Ausgangsstromes, enthaltend einen oder mehrere Kohlenwasserstoffe, mit einer vorgegebenen Konzentration an Sauerstoff und einer vorgegebenen Temperatur, eine Vorrichtung zur Durchführung des Verfahrens sowie eine Verwendung.

Es ist häufig erforderlich, für den Einsatz in Verfahren zu endothermen Umsetzungen einen Sauerstoff enthaltenden Ausgangsstrom bereitzustellen, der eine vorgegebene Konzentration an Sauerstoff und eine vorgegebene Temperatur aufweist. Die benötigten Temperaturen sind häufig so hoch, dass sie nicht allein durch Wärmeintegration mit den verfahrenseigenen Produktströmen erreicht werden können. In Verfahren nach dem Stand der Technik wird daher häufig auf eine insbesondere für Großanlagen sehr kostenintensive elektrische Aufheizung des Sauerstoff enthaltenden Einsatzgasstromes zurückgegriffen. Die elektrische Aufheizung hat den weiteren Nachteil, dass sie insbesondere schlecht regelbar ist.

DE 10 2004 024 957 offenbart ein Verfahren für die Oxidehydrierung von Propan oder von Butan. Die Zuführung eines zweiten Luftstromes oder die Steuerung eines Ausgangsstromes durch die Sauerstoffmenge oder die Temperatur des zweiten Luftstromes sind in DE 10 2004 024 957 nicht offenbart.

Es war daher Aufgabe der Erfindung, ein Verfahren und eine Vorrichtung zur Bereitstellung eines Sauerstoff enthaltenden Einsatzgasstromes mit vorgegebener Konzentration an Sauerstoff und vorgegebener Temperatur zur Verfügung zu stellen, das technisch einfach durchführbar und im Vergleich zu einer elektrischen Aufheizung weniger aufwändig ist, und das eine gezielte Einstellung des Sauerstoffgehaltes und der Temperatur ermöglicht.

Die Aufgabe wird gelöst durch ein Verfahren zur Bereitstellung eines Sauerstoff enthaltenden Gasstromes für die endotherme Umsetzung eines Ausgangsstromes, enthaltend einen oder mehrere Kohlenwasserstoffe, mit einer vorgegebenen Konzentration an Sauerstoff und einer vorgegebenen Temperatur, das dadurch gekennzeichnet ist, dass ein fluider Brennstoffstrom
mit einem Primärluftstrom bei λ-Werten des Primärluftstromes zum fluiden Brennstoffstrom von 0,6 bis 1,2 unter Erhalt eines Verbrennungsgasstromes verbrannt wird,
und zum Verbrennungsgasstrom ein Sekundärluftstrom zugemischt wird, unter Erhalt des Sauerstoff enthaltenden Gasstromes für die endotherme Umsetzung,
wobei über den Mengenstrom und die Temperatur des Sekundärluftstromes die vorgegebene Konzentration an Sauerstoff sowie die vorgegebene Temperatur des Sauerstoff enthaltenden Gasstromes eingestellt werden,
und wobei die endotherme Umsetzung die Oxidehydrierung von Propan oder von Butan ist.

Es wurde gefunden, dass es möglich ist, durch Verbrennung eines fluiden Brennstoffstroms mit einem ersten, so genannten Primärluftstrom und anschließender Zumischung eines weiteren, so genannten Sekundärluftstromes, einen Gasstrom für die endotherme Umsetzung zur Verfügung zu stellen, der die für eine vorgegebene endotherme Umsetzung erforderlichen Voraussetzungen bezüglich der Konzentration an Sauerstoff und bezüglich der Temperatur erfüllt. Der bereitgestellte, Sauerstoff enthaltende Gasstrom ist ein direktes Oxidationsmittel zur Abdeckung des Wärmebedarfes für die vorgegebene endotherme Umsetzung.

Der Begriff Fluid bezeichnet in bekannte Weise alle Flüssigkeiten, Dämpfe und Gase, die den strömungstechnischen Gesetzen nicht fester Kontinua folgen. Der fluide Brennstoffstrom kann insbesondere ein gasförmiger Brennstoffstrom sein.

Besonders vorteilhaft kann der fluide Brennstoffstrom einen Teilstrom des, für das nachfolgende Verfahren der endothermen Umsetzung bereits in der Anlage vorhandenen Ausgangsstromes, enthaltend einen oder mehrere Kohlenwasserstoffe, sein.

Vorteilhaft kann dem fluiden Brennstoffstrom, der ein Teilstrom des, für das nachfolgende Verfahren der endothermen Umsetzung bereits in der Anlage vorhandenen Ausgangsstromes, enthaltend einen oder mehrere Kohlenwasserstoffe, ist, zusätzlich ein Methan enthaltendes Gas zugemischt werden.

Weiterhin kann dem fluiden Brennstoffstrom, der ein Teilstrom des einen oder mehrere Kohlenwasserstoffe enthaltenden Ausgangsstromes der endothermen Umsetzung ist, zusätzlich ein Rückführstrom aus der endothermen Umsetzung zugemischt werden.

Die endotherme Umsetzung unter Verwendung eines Sauerstoff enthaltenden Gasstromes ist die Oxidehydrierung von Propan oder von Butan.

Entsprechend enthält der Ausgangsstrom Propan oder Butan.

Der Anteil des in der nachfolgenden endothermen Umsetzung einzusetzenden fluiden Brennstoffstroms bestimmt sich aus den konkreten Anforderungen der durchzuführenden Umsetzung, insbesondere der Temperatur, die der Sauerstoff enthaltende Gasstrom bei der Zuführung in die endotherme Umsetzung aufweisen muss. Diese Temperatur kann häufig im Bereich von 400 bis 700°C liegen.

Der Begriff λ-Wert wird in bekannter Weise zur Charakterisierung von Gemischzusammensetzungen, enthaltend einen oder mehrere Brennstoffe, insbesondere Kohlenwasserstoffe und Luft benutzt, und wird definiert als das tatsächliche Luft- zu Brennstoffverhältnis, bezogen auf das stöchiometrische Luft- zu Brennstoffverhältnis. Ein λ-Wert von 1 entspricht somit der Zusammensetzung für eine vollständige Verbrennung, λ-Werte von größer als 1 entsprechen mageren und λ-Werte von kleiner als 1 fetten Luft/Brennstoff-Gemischen.

Nach dem erfindungsgemäßen Verfahren wird der λ-Wert des Gemisches aus dem fluiden Brennstoffstrom und dem Primärluftstrom auf einen λ-Wert im Bereich von 0,6 bis 1,2 eingestellt, das heißt das Gemisch wird leicht fett bis leicht mager eingestellt. Entsprechend kann der Verbrennungsgasstrom noch Restkohlenwasserstoffe enthalten. Diese werden gegebenenfalls im nachfolgenden Verfahrensschritt durch Zumischen des Sekundärluftstromes vollständig verbrannt. Bevorzugt kann der Verbrennungsgasstrom jedoch noch einen Überschuss an unverbrauchtem Sauerstoff, insbesondere im Bereich von 2 bis 3 Vol-%, bezogen auf das Gesamtvolumen des Verbrennungsgasstromes, enthalten.

Die Verbrennung des Teilstromes des Ausgangsstromes mit dem Primärluftstrom erfolgt insbesondere bei einer Temperatur im Bereich von 1500 bis 1700°C.

Dem Verbrennungsgasstrom wird anschließend ein Sekundärluftstrom zugemischt, der bevorzugt Wasserdampf enthält, um die Rußbildung bei der Verbrennung zu unterdrücken.

Der Anteil des zugesetzten Wasserdampfes im Sekundärluftstrom wird bevorzugt so gewählt, dass im Austrittsgasstrom, das heißt im Sauerstoff enthaltenden Gasstrom, der in der endothermen Umsetzung verwendet wird, der Wasserdampfgehalt im Bereich von 0 bis 50 Vol-%, insbesondere im Bereich von 0,5 bis 4 Vol-%, liegt.

Bevorzugt werden der Primärluftstrom und der Sekundärluftstrom vorgewärmt, insbesondere auf eine Temperatur im Bereich 150 bis 200°C, bevorzugt auf etwa 170°C.

Bevorzugt kann die Aufheizung des Primärluftstromes und/oder des Sekundärluftstromes durch Wärmeintegration mit dem Produktstrom der endothermen Umsetzung durchgeführt werden.

Der Sauerstoffgehalt des durch das Verfahren bereitgestellte Sauerstoff enthaltenden Stromes wird, inbesondere durch eine entsprechende Steuerung der zugegebenen Menge an Wasserdampf und insbesondere Sekundärluft, bevorzugt auf einen Wert im Bereich zwischen 2 und 20 Vol-%, weiter bevorzugt zwischen 13 und 20 Vol-%, eingestellt.

Gegenstand der Erfindung ist auch eine Vorrichtung zur Durchführung des obigen Verfahrens zur Bereitstellung einer Sauerstoff enthaltenden Gasstromes für die endotherme Umsetzung eines Ausgangsstromes, enthaltend Propan oder Butan, mit einer vorgegebenen Konzentration an Sauerstoff und einer vorgegebenen Temperatur, wobei ein fluider Brennstoffstrom mit einem Primärluftstrom bei λ-Werten des fluiden Brennstoffstroms von 0,6 bis 1,2 unter Erhalt eines Verbrennungsgasstromes vollständig verbrannt wird,
und zum Verbrennungsgasstrom ein Sekundärluftstrom zugemischt wird, unter Erhalt des Sauerstoff enthaltenden Gasstromes für die endotherme Umsetzung,
wobei über den Mengenstrom und die Temperatur des Sekundärluftstromes die vorgegebene Konzentration an Sauerstoff sowie die vorgegebene Temperatur des Sauerstoff enthaltenden Gasstromes eingestellt werden, die
durch eine lang gestreckte Brennkammer gekennzeichnet ist,
mit einer Zuführung des fluiden Brennstoffstromes über eine oder mehrere, zentral, an einem Ende der Brennkammer angeordnete Düsen, die einen Brenner bilden, und des Primärluftstromes in den Bereich der Düsen,
einer Zuführung des Sekundärluftstromes über Öffnungen am Umfang der Brennkammer, sowie
mit einer Austrittsöffnung für den durch das Verfahren bereitgestellte Sauerstoff enthaltenden Gasstrom am anderen Ende der Brennkammer.

Die Vorrichtung umfasst eine lang gestreckte Brennkammer, die aus einem hochtemperaturbeständigen Werkstoff, insbesondere einem hochtemperaturbeständigen Stahl, bevorzugt einem Chrom-Nickel-Stahl gebildet ist, und die mit einer äußeren Wärmeisolierung versehen ist.

Die Brennkammer kann bevorzugt aufrecht stehend angeordnet sein, mit dem brennerseitigen Ende unten und dem austrittsgasseitigen Ende oben.

Die lang gestreckte Brennkammer weist an einem Ende derselben eine Zuführung für den fluiden Brennstoffstrom auf, die zentral angeordnet ist und die in die Brennkammer über eine oder mehrere Düsen mündet, die einen Brenner bilden.

In den Bereich der Düsen wird der Primäriuftstrom eingeleitet, insbesondere über eine Leitung, die konzentrisch um die Zuleitung für den fluiden Brennstoffstrom angeordnet ist.

Die Verbrennung wird über einen Flammenwächter überdacht, wie er in der Brennertechnik gebräuchlich ist.

Für die Zumischung von Sekundärluft zu den Verbrennungsgasen aus dem Brenner sind Öffnungen am Umfang der Brennkammer vorgesehen.

Die Öffnungen am Umfang der Brennkammer für die Zuführung des Sekundärluftstromes sind in Form von lamellenartigen, ringförmigen Schlitzen ausgebildet. Diese sind bevorzugt ab der Mitte der Brennkammer angeordnet und werden dadurch gebildet, dass die Brennkammerwand aus zwei oder mehreren, konzentrischen Rohrabschnitten gebildet ist, mit zunehmendem Durchmesser bei zunehmendem Abstand der Rohrabschnitte vom Brenner und wobei der Außendurchmesser des vorhergehenden Rohrabschnittes kleiner ist als der Innendurchmesser des jeweils nachfolgenden Rohrabschnittes. Dadurch verbleiben zwischen den, teilweise ineinander geschobenen Rohrabschnitten, die ringförmigen Schlitze für die Zuführung des Sekundärluftstromes.

Durch die besondere Ausführung mit den oben beschriebenen ringförmigen Schlitzen für die Zuführung des Sekundärluftstromes wird nicht nur die Einstellung des vorgegebenen Sauerstoffgehaltes sowie der vorgegebenen Temperatur für den bereitzustellenden, Sauerstoff enthaltenden Gasstrom, erreicht, der Sekundärluftstrom hat darüber hinaus eine Kühlwirkung für die Wand der Brennkammer.

Am Ende der Brennkammer, das dem Brenner gegenüberliegt, befindet sich eine Austrittsöffnung für den Sauerstoff enthaltenden Gasstrom. Im Bereich der Austrittsöffnung kann bevorzugt ein statischer Mischer angeordnet sein.

Die Erfindung wird im Folgenden anhand einer Zeichnung näher erläutert.

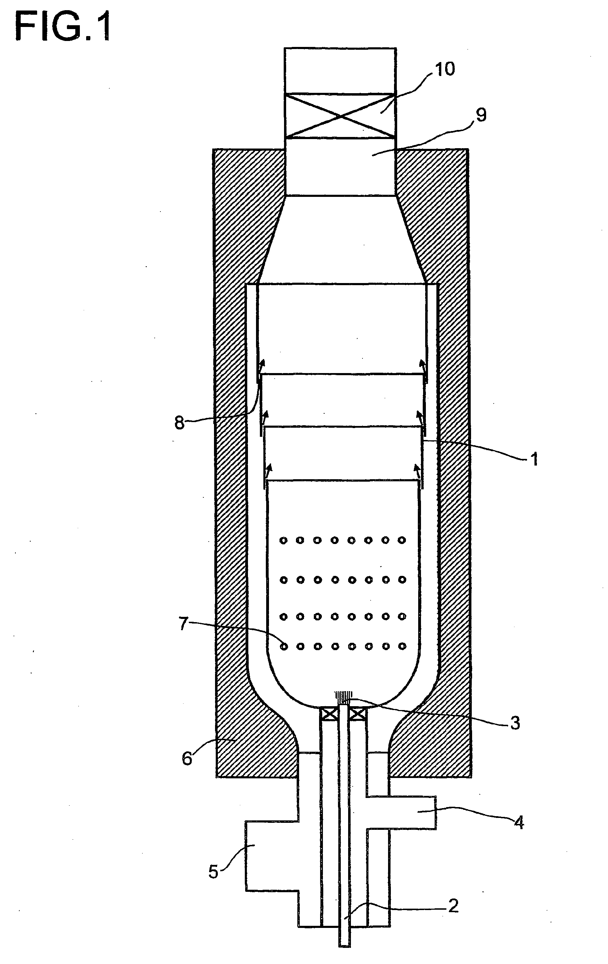

Die einzige Figur 1 zeigt eine schematische Schnittdarstellung durch eine bevorzugte Ausführungsform für eine erfindungsgemäße Brennkammer.

Die Brennkammer 1 weist an einem Ende eine Zuführung 2 für den fluiden Brennstoffstrom auf, die in einer oder mehreren Düsen 3 in der Brennkammer 1 endet, sowie eine Zuführung 4 für den Primärluftstrom, die konzentrisch um die Zuführung 2 für den Ausgangsstrom angeordnet ist und die im Bereich der Düsen 3 mündet.

Der Sekundärluftstrom wird über eine weitere Zuführung 5 zwischen der äußeren Wand der Brennkammer 1 und der Isolation 6 in die Brennkammer geleitet und kann über Perforationen 7, die auf beispielhaft vier ringförmigen Umfangbahnen angeordnet sind, in die Brennkammer 1 eintreten.

Ab etwa der Mitte der Brennkammer 1 sind beispielhaft drei ringförmige Schlitze 8 vorgesehen, über die die Sekundärluft entlang der Brennkammerwand in die Brennkammer 1 einströmt.

Die Brennkammer mündet in eine Austrittsöffnung 9 für den Sauerstoff enthaltenden Gasstrom. Im Bereich der Austrittsöffnung 9 ist ein statischer Mischer 10 angeordnet.

## Patentansprüche

1. Verfahren zur endothermen Umsetzung eines Ausgangsstromes unter Verwendung eines Sauerstoff enthaltenden Gasstromes, enthaltend einen oder mehrere Kohlenwasserstoffe, mit einer vorgegebenen Konzentration an Sauerstoff und einer vorgegebenen Temperatur, wobei ein fluider Brennstoffstrom mit einem Primärluftstrom bei λ-Werten des Primärluftstromes zum fluiden Brennstoffstrom von 0,6 bis 1,2, unter Erhalt eines Verbrennungsgasstromes verbrannt wird,
und zum Verbrennungsgasstrom ein Sekundärluftstrom zugemischt wird, unter Erhalt des Sauerstoff enthaltenden Gasstromes für die endotherme Umsetzung, wobei über den Mengenstrom und die Temperatur des Sekundärluftstromes die vorgegebene Konzentration an Sauerstoff im Bereich von 2 bis 20 Vol.-% sowie die vorgegebene Temperatur des Sauerstoff enthaltenden Gasstromes eingestellt werden, und wobei
die endotherme Umsetzung die Oxidehydrierung von Propan oder von Butan ist.

2. Verfahren nach Anspruch 1, **dadurch gekennzeichnet, dass** der Sekundärluftstrom Wasserdampf enthält.

3. Verfahren nach Anspruch 1 oder 2, **dadurch gekennzeichnet, dass** der fluide Brenstoffstrom ein Teilstrom des einen oder mehrere Kohlenwasserstoffe enthaltenden Ausgangsstromes ist.

4. Verfahren nach Anspruch 3, **dadurch gekennzeichnet, dass** dem fluiden Brennstoffstrom zusätzlich ein Methan enthaltendes Gas zugemischt wird.

5. Verfahren nach Anspruch 3 oder 4, **dadurch gekennzeichnet, dass** dem fluiden Brennstoffstrom zusätzlich ein Rückführstrom aus der endothermen Umsetzung zugemischt wird.

6. Verfahren nach einem der Ansprüche 1 bis 5, **dadurch gekennzeichnet, dass** der fluide Brennstoffstrom mit dem Primärluftstrom bei einem λ-Wert von 0,8 verbrannt wird.

7. Verfahren nach einem der Ansprüche 1 bis 6, **dadurch gekennzeichnet, dass** der Primärluftstrom und/oder der Sekundärluftstrom durch Wärmeintegration mit dem Produktstrom der endothermen Umsetzung vorerwärmt wird.

8. Verfahren nach Anspruch 7, **dadurch gekennzeichnet, dass** die Vorerwärmung des Primärluftstromes und/oder des Sekundärluftstromes auf eine Temperatur zwischen 150 und 200°C, bevorzugt auf 170°C, erfolgt.

9. Verfahren nach einem der Ansprüche 1 bis 8, **dadurch gekennzeichnet, dass** der Verbrennungsgasstrom 2 bis 3 Vol-% Restsauerstoff enthält.

10. Verfahren nach einem der Ansprüche 2 bis 9, **dadurch gekennzeichnet, dass** der Wasserdampfgehalt im Sekundärluftstrom dergestalt eingestellt wird, dass der durch das Verfahren bereitgestellte, Sauerstoff enthaltende Gasstrom einen Wasserdampfgehalt im Bereich von 0 bis 50 Vol-%, insbesondere im Bereich von 0,5 bis 4 Vol-%, aufweist.

11. Verfahren nach einem der Ansprüche 1 bis 10, **dadurch gekennzeichnet, dass** der Sauerstoffgehalt im durch das Verfahren bereitgestellten, Sauerstoff enthaltenden Gasstrom auf 13 bis 20 Vol-%, eingestellt wird.

12. Vorrichtung zur Durchführung des Verfahrens nach einem der Ansprüche 1 bis 11 zur endothermen Umsetzung eines Ausgangsstromes, wobei die endotherme Umsetzung die Oxidehydrierung von Propan oder von Butan ist, unter Verwendung einer Sauerstoff enthaltenden Gasstromes, enthaltend einen oder mehrere Kohlenwasserstoffe, mit einer vorgegebenen Konzentration an Sauerstoff und einer vorgegebenen Temperatur, wobei ein fluider Brennstoffstrom mit einem Primärluftstrom bei λ-Werten des Primärluftstromes zum fluiden Brennstoffstrom von 0,6 bis 1,2 unter Erhalt eines Verbrennungsgasstromes verbrannt wird,
und zum Verbrennungsgasstrom ein Sekundärluftstrom zugemischt wird, unter Erhalt des Sauerstoff enthaltenden Gasstromes mit einem Sauerstoffgehalt von 2 bis 20 Vol.-% für die endotherme Umsetzung,
wobei über den Mengenstrom und die Temperatur des Sekundärluftstromes die vorgegebene Konzentration an Sauerstoff sowie die vorgegebene Temperatur des Sauerstoff enthaltenden Gasstromes eingestellt werden,
**gekennzeichnet durch** eine lang gestreckte Brennkammer (1),
mit einer Zuführung (2) des Teilstromes des fluiden Brennstoffstromes über eine oder mehrere, zentral, an einem Ende der Brennkammer angeordnete Düsen (3), die einen Brenner bilden, einer Zuführung (4) des Primärluftstromes in den Bereich der Düsen (3), und einer Zuführung (5) des Sekundärluftstromes über Öffnungen am Umfang der Brennkammer (1), sowie
mit einer Austrittsöffnung (9) für den Sauerstoff enthaltenden Gasstrom am anderen Ende der Brennkammer (1), und wobei die Öffnungen am Umfang der Brennkammer (1) für die Zuführung des Sekundärluftstromes in Form eines oder mehrere lamellenartiger, ringförmiger Schlitze vorgesehen sind, die **dadurch** gebildet werden, dass die Wand der Brennkammer (1) aus zwei oder mehreren Rohrabschnitten gebildet ist, die mit zunehmendem Abstand vom brennerseitigen Ende der Brennkammer (1) einen zunehmenden Innendurchmesser aufweisen, und die teilweise ineinander geschoben sind, wobei jedoch der Außendurchmesser des vorangehenden Rohrabschnittes kleiner ist als der Innendurchmesser des nachfolgenden Rohrabschnittes, und wodurch der eine oder die mehreren ringförmigen Schlitze (8) ausgebildet werden.

13. Vorrichtung nach Anspruch 12, **dadurch gekennzeichnet, dass** im Bereich der Austrittsöffnung (9) für den Sauerstoff enthaltenden Gasstrom ein statischer Mischer (10) angeordnet ist.

14. Vorrichtung nach Anspruch 12 oder 13, **dadurch gekennzeichnet, dass** die Brennkammer (1) aus einem hochtemperaturbeständigen Stahl, insbesondere einem Chrom-Nickel-Stahl, gebildet ist.

## Claims

1. A method for the endothermic reaction of an initial stream using an oxygen-comprising gas stream, comprising one or more hydrocarbons, having a predetermined oxygen concentration and a predetermined temperature, where a fluid fuel stream
is combusted with a primary air stream at λ values of the primary air stream to the fluid fuel stream of from 0.6 to 1.2 to obtain a combustion gas stream,
and a secondary air stream is admixed to the combustion gas stream to obtain the oxygen-comprising gas stream for the endothermic reaction,
with the predetermined oxygen concentration in the range of from 2 to 20% by volume and the predetermined temperature of the oxygen-comprising gas stream being adjusted via the flow rate and the temperature of the secondary air stream, and with
the endothermic reaction being the oxydehydration of propane or of butane.

2. The method according to claim 1, wherein the secondary air stream comprises water vapor.

3. The method according to claim 1 or 2, wherein the fluid fuel stream is a partial stream of the initial stream comprising one or more hydrocarbons.

4. The method according to claim 3, wherein the fluid fuel stream additionally has a methane-comprising gas admixed thereto.

5. The method according to claim 3 or 4, wherein the fluid fuel stream additionally has a return stream from the endothermic reaction admixed thereto.

6. The method according to one of claims 1 to 5, wherein the fluid fuel stream is combusted with the primary air stream at a λ value of 0.8.

7. The method according to one of claims 1 to 6, wherein the primary air stream and/or the secondary air stream is preheated by heat integration with the product stream from the endothermic reaction.

8. The method according to claim 7, wherein the preheating of the primary air stream and/or of the secondary air stream is carried out to a temperature of between 150 and 200 °C, preferably to 170 °C.

9. The method according to one of claims 1 to 8, wherein the combustion gas stream comprises 2 to 3% by volume of residual oxygen.

10. The method according to one of claims 2 to 9, wherein the water vapor content in the secondary air stream is adjusted in such a way that the oxygen-comprising gas stream provided by the method has a water vapor content in the range of from 0 to 50% by volume, in particular in the range of from 0.5 to 4% by volume.

11. The method according to one of claims 1 to 10, wherein the oxygen content in the oxygen-comprising gas stream provided by the method is adjusted to 13 to 20% by volume.

12. A device for carrying out the method according to one of claims 1 to 11 for the endothermic reaction of an initial stream, where the endothermic reaction is the oxydehydration of propane or of butane, using an oxygen-comprising gas stream, comprising one or more hydrocarbons, having a predetermined oxygen concentration and a predetermined temperature, wherein a fluid fuel stream is combusted with a primary air stream at λ values of the primary air stream to the fluid fuel stream of from 0.6 to 1.2 to obtain a combustion gas stream,
and a secondary air stream is admixed to the combustion gas stream to obtain the oxygen-comprising gas stream having an oxygen content of from 2 to 20% by volume for the endothermic reaction,
with the predetermined oxygen concentration and the predetermined temperature of the oxygen-comprising gas stream being adjusted via the flow rate and the temperature of the secondary air stream,
**characterized by** an elongated combustion chamber (1)
having a supply (2) of the partial stream of the fluid fuel stream by means of one or more nozzles (3) which are centrally arranged at one end of the combustion chamber to form a burner, a supply (4) of the primary air stream into the nozzle (3) region,
and a supply (5) of the secondary air stream by means of openings on the perimeter of the combustion chamber (1), and
having an outlet opening (9) for the oxygen-comprising gas stream at the other end of the combustion chamber (1), and with the openings on the perimeter of the combustion chamber (1) for the supply of the secondary air stream being provided in form of one or more fin-like annular slots, which are formed as a result of the wall of the combustion chamber (1) being comprised of two or more tube sections, the inner diameter of which increases with increasing distance from the burner end of the combustion chamber (1), and which are partly inserted into one another, wherein, however, the outer diameter of the preceding tube section is smaller than the inner diameter of the subsequent tube section, and which results in the formation of the one or more annular slots (8).

13. The device according to claim 12, wherein a static mixer (10) is disposed in the region of the outlet opening (9) for the oxygen-comprising gas stream.

14. The device according to claim 12 or 13, wherein the combustion chamber (1) is made of a high temperature resistant steel, in particular a chromium-nickel steel.

## Revendications

1. Procédé de réaction endothermique d'un courant initial utilisant un courant gazeux contenant de l'oxygène, contenant un ou plusieurs hydrocarbures, ayant une concentration prédéterminée en oxygène et une température prédéterminée, selon lequel un courant de combustible fluide
est brûlé avec un courant d'air primaire à des valeurs λ entre le courant d'air primaire et le courant de combustible fluide de 0,6 à 1,2, pour obtenir un courant de gaz de combustion,
et un courant d'air secondaire est mélangé avec le courant de gaz de combustion, pour obtenir le courant gazeux contenant de l'oxygène pour la réaction endothermique,
la concentration prédéterminée en oxygène dans la plage allant de 2 à 20 % en volume, ainsi que la température prédéterminée du courant gazeux contenant de l'oxygène étant ajustées par le biais du débit massique et de la température du courant d'air secondaire, et
la réaction endothermique étant l'oxydéshydrogénation de propane ou de butane.

2. Procédé selon la revendication 1, **caractérisé en ce que** le courant d'air secondaire contient de la vapeur d'eau.

3. Procédé selon la revendication 1 ou 2, **caractérisé en ce que** le courant de combustible fluide est un courant partiel du courant initial contenant un ou plusieurs hydrocarbures.

4. Procédé selon la revendication 3, **caractérisé en ce qu'**un gaz contenant du méthane est également mélangé avec le courant de combustible fluide.

5. Procédé selon la revendication 3 ou 4, **caractérisé en ce qu'**un courant recyclé issu de la réaction endothermique est également mélangé avec le courant de combustible fluide.

6. Procédé selon l'une quelconque des revendications 1 à 5, **caractérisé en ce que** le courant de combustible fluide est brûlé avec le courant d'air primaire à une valeur λ de 0,8.

7. Procédé selon l'une quelconque des revendications 1 à 6, **caractérisé en ce que** le courant d'air primaire et/ou le courant d'air secondaire sont préchauffés par intégration thermique avec le courant de produits de la réaction endothermique.

8. Procédé selon la revendication 7, **caractérisé en ce que** le préchauffage du courant d'air primaire et/ou du courant d'air secondaire a lieu à une température comprise entre 150 et 200 °C, de préférence à 170 °C.

9. Procédé selon l'une quelconque des revendications 1 à 8, **caractérisé en ce que** le courant de gaz de combustion contient 2 à 3 % en volume d'oxygène résiduel.

10. Procédé selon l'une quelconque des revendications 2 à 9, **caractérisé en ce que** la teneur en vapeur d'eau du courant d'air secondaire est ajustée de manière à ce que le courant gazeux contenant de l'oxygène préparé par le procédé présente une teneur en vapeur d'eau dans la plage allant de 0 à 50 % en volume, notamment dans la plage allant de 0,5 à 4 % en volume.

11. Procédé selon l'une quelconque des revendications 1 à 10, **caractérisé en ce que** la teneur en oxygène du courant gazeux contenant de l'oxygène préparé par le procédé est ajustée à 13 à 20 % en volume.

12. Dispositif pour la réalisation du procédé selon l'une quelconque des revendications 1 à 11 pour la réaction endothermique d'un courant initial, la réaction endothermique étant l'oxydéshydrogénation de propane ou de butane, utilisant un courant gazeux contenant de l'oxygène, contenant un ou plusieurs hydrocarbures, ayant une concentration prédéterminée en oxygène et une température prédéterminée, selon lequel un courant de combustible fluide est brûlé avec un courant d'air primaire à des valeurs λ entre le courant d'air primaire et le courant de combustible fluide de 0,6 à 1,2, pour obtenir un courant de gaz de combustion, et un courant d'air secondaire est mélangé avec le courant de gaz de combustion, pour obtenir le courant gazeux contenant de l'oxygène ayant une teneur en oxygène de 2 à 20 % en volume pour la réaction endothermique,
la concentration prédéterminée en oxygène, ainsi que la température prédéterminée du courant gazeux contenant de l'oxygène étant ajustées par le biais du débit massique et de la température du courant d'air secondaire,
**caractérisé par** une chambre de combustion (1) étendue longitudinalement,
comprenant une alimentation (2) du courant partiel du courant de combustible fluide par une ou plusieurs buses (3) agencées centralement à une extrémité de la chambre de combustion, qui forment un brûleur, une alimentation (4) du courant d'air primaire dans la zone des buses (3), et une alimentation (5) du courant d'air secondaire par des ouvertures sur la périphérie de la chambre de combustion (1), ainsi que
comprenant une ouverture de sortie (9) pour le courant gazeux contenant de l'oxygène à l'autre extrémité de la chambre de combustion (1), les ouvertures sur la périphérie de la chambre de combustion (1) pour l'alimentation du courant d'air secondaire étant prévues sous la forme d'une ou de plusieurs fentes annulaires de type lamellaire, qui sont formées en ce que la paroi de la chambre de combustion (1) est formée de deux sections tubulaires ou plus, qui présentent un diamètre intérieur croissant à une distance croissante de l'extrémité du brûleur de la chambre de combustion (1) et qui sont partiellement enfilées les unes dans les autres, le diamètre extérieur de la section tubulaire précédente étant toutefois inférieur au diamètre intérieur de la section tubulaire ultérieure, et la ou les fentes annulaires (8) étant ainsi formées.

13. Dispositif selon la revendication 12, **caractérisé en ce qu'**un mélangeur statique (10) est agencé dans la zone de l'ouverture de sortie (9) pour le courant gazeux contenant de l'oxygène.

14. Dispositif selon la revendication 12 ou 13, **caractérisé en ce que** la chambre de combustion (1) est formée en un acier résistant aux températures élevées, notamment en un acier chrome-nickel.
